# DEMANDE DE BREVET EUROPEEN

(11) **EP 2 886 143 A1**
(43) Date de publication de la demande: **24.06.2015**
(21) Numéro de dépôt: 14199735.3
(22) Date de dépôt: 22.12.2014
(51) Int. Cl.: A61M 5/158, A61M 25/06, A61M 39/02

(54) **Dispositif de perfusion ou d'injection sécurisé**

(30) Priorité: 23.12.2013 FR 1363496
(71) Demandeur: Vygon, 95440 Ecouen (FR)
(72) Inventeur: Carrez, Jean Luc, 95440 Ecouen (FR)
(74) Mandataire: Regimbeau

(57) **Abrégé**

L'invention concerne un dispositif de perfusion ou d'injection sécurisé (1), notamment pour la perfusion ou l'injection d'un fluide dans une chambre implantable (5), comprenant :
- une aiguille (3) pleine présentant une extrémité distale biseautée (3c), et
- une embase (2), comprenant
* un raccord principal (10), configuré pour être appliqué contre la peau du patient et formant un passage principal (12) adapté pour recevoir ladite aiguille (3) à coulissement,
* une canule (20) s'étendant depuis l'embase (2) dans le prolongement du passage principal (12), ladite canule (20) étant configurée pour recevoir l'aiguille (3) à coulissement.

## Description

### DOMAINE DE L'INVENTION

L'invention concerne un dispositif de perfusion ou d'injection sécurisé, notamment pour la perfusion ou l'injection d'un fluide dans une chambre implantable.

### ARRIERE-PLAN TECHNOLOGIQUE

Une chambre implantable est un réservoir d'accès vasculaire central qui peut être implanté sous la peau d'un patient afin de perfuser ou d'injecter des fluides comprenant généralement des médicaments. Une chambre implantable permet un accès répété au système vasculaire du patient en réduisant le risque infectieux. Elle comprend à cet effet une chambre (ou réservoir) dont une extrémité ouverte est obturée par une membrane pouvant être en silicone à travers lequel sont réalisées les injections, un cathéter pour relier la chambre au réseau veineux, et un système de verrouillage du cathéter à la chambre.

Des aiguilles de Huber sont généralement utilisées pour la perfusion et l'injection de fluides dans les chambres implantables. De manière connue en soi, une aiguille de Huber comprend une embase Luer ou Luer Lock et une canule droite ou coudée à 90°, munie au niveau de son extrémité distale d'un biseau tangentiel spécifique dit de Huber, qui permet de nombreuses perforation de la membrane tout en limitant les dommages qui lui sont causés et la douleur du patient au moment de l'introduction de l'aiguille de Huber. A titre d'exemple, une membrane doit recevoir de l'ordre de 3500 ponctions et doit donc assurer une bonne obturation automatique, ce qui implique pour l'opérateur de fournir un effort important pour introduire et retirer l'aiguille dans la membrane. Ces efforts nécessitent généralement à l'opérateur de prendre appui sur la peau du patient pour maintenir la chambre implantable en position. Or, compte tenu des efforts importants en jeu, il existe un risque pour l'opérateur de se blesser avec l'aiguille en se piquant un doigt.

On a donc proposé des systèmes permettant de manipuler en sécurité des aiguilles telles que l'aiguille de Huber afin de perfuser d'une chambre implantable. Par exemple, le document FR 2 847 478 décrit un dispositif anti-pique conformé de manière à faciliter l'application du dispositif sur la peau du patient, et comprenant des moyens permettant d'extraire l'aiguille facilement et en supprimant le risque d'accidents avec exposition au sang. En particulier, l'aiguille est automatiquement dirigée vers un piège de sécurité.

Néanmoins, la Demanderesse s'est aperçue que les aiguilles généralement utilisées pour la perfusion et l'injection de produits dans les chambres implantables, telles que les aiguilles de Huber, avaient tout de même tendance à abîmer la membrane. En effet, bien que la forme en biseau tangentiel permette de limiter les dommages causés à la membrane, il n'en reste pas moins que le biseau a tendance à le carotter légèrement, de sorte que la répétition des ponctions dans la membrane l'endommage progressivement. Par ailleurs, de telles aiguilles représentent une partie importante du coût des dispositifs actuellement utilisés. Elles peuvent en outre s'avérer difficiles à obtenir. Par ailleurs, leur biseau est relativement fragile et s'abîme facilement. Or, généralement, l'utilisateur pousse l'aiguille en butée contre le fond de la chambre pour entendre le clic et s'assurer d'avoir introduit l'aiguille jusqu'au fond de la chambre, ce qui écorne la pointe du biseau, qui est fragile, et a pour conséquence de labourer le septum lors de son retrait en l'endommageant.

Enfin, la forme coudée, qui est la forme la plus utilisée dans le cas des chambres implantables, s'avère peu ergonomique et difficile à manipuler par les opérateurs.

### RESUME DE L'INVENTION

Un objectif de l'invention est de proposer une solution aux problèmes exposés ci-dessus. Plus particulièrement, un objectif de l'invention est de proposer un nouveau dispositif de perfusion ou d'injection sécurisé, notamment pour la perfusion ou l'injection d'un fluide dans une chambre implantable, qui soit plus ergonomique, facile à industrialiser, moins fragile, et qui réduise l'endommagement d'une membrane lors de l'introduction et du retrait de l'aiguille dans une chambre implantable afin de prolonger la durée de vie desdites chambres implantables.

Pour cela, l'invention propose un dispositif de perfusion ou d'injection sécurisé, notamment pour la perfusion ou l'injection d'un fluide dans une chambre implantable, comprenant :
- une aiguille pleine présentant une extrémité distale biseautée (3c), et
- une embase, comprenant
   * un raccord principal, configuré pour être appliqué contre la peau du patient et formant un passage principal adapté pour recevoir ladite aiguille à coulissement,
   * une canule s'étendant depuis l'embase dans le prolongement du passage principal, ladite canule étant configurée pour recevoir l'aiguille à coulissement.

Certaines caractéristiques préférées mais non limitatives du dispositif décrit ci-dessus sont les suivantes :
- le dispositif comprend en outre un tube, fixé sur l'embase et en communication fluidique avec le passage principal et la canule,
- la différence de diamètre entre la canule et l'aiguille est comprise entre 0.05 et 0.005 mm, par exemple de l'ordre de 0.01 mm,
- une extrémité distale de la canule comprend un biseau émoussé formant un angle compris entre 5° et 20°, par exemple de l'ordre de 10°,
- le raccord principal comprend en outre un septum logé dans le passage principal et configuré pour obturer ledit passage principal, et un capot configuré pour comprimer le septum dans le raccord principal,
- l'embase comprend en outre une cage anti-pique, fixée de manière amovible sur une extrémité proximale du raccord principal, ladite cage anti-pique comprenant un corps creux définissant un passage pour l'aiguille et comprenant des organes de verrouillage amovibles, adaptés pour venir en prise avec des organes de verrouillage associés formés sur l'embase, et un organe de retenue anti-pique, comprenant une rondelle fixée dans le passage du corps creux et adaptée pour venir en prise avec un organe associé de l'aiguille,
- l'organe associé de l'aiguille comprend une rainure circonférentielle, et l'organe de retenue anti-pique est disposé au niveau d'une extrémité proximale du corps creux et comprend des pattes adaptée pour venir se loger dans la rainure circonférentielle et limiter le coulissement de l'aiguille par rapport au corps creux,
- l'organe de retenue est en métal et est obtenu par découpage à l'aide d'un poinçon et d'une matrice de sorte que ledit organe de retenue présente une première face dont des bords sont arrondis et une deuxième face dont des bords sont tranchants, la première face étant configurée pour s'étendre en regard de la canule,
- le corps creux comprend en outre une paroi centrale, s'étendant sensiblement perpendiculairement à une direction de coulissement de l'aiguille, ladite paroi centrale présentant en son centre un orifice traversant pour guider l'aiguille,
- les organes de verrouillage amovibles sont du type à baïonnette,
- l'aiguille comprend, au niveau d'une extrémité proximale, un organe de manipulation solidaire en mouvement de ladite aiguille et adapté pour venir en butée contre l'embase lorsque l'aiguille est introduite dans le passage principal dudit raccord principal,
- la canule est configurée de sorte que, lorsque l'organe de manipulation est en butée contre l'embase, une distance entre une extrémité distale de la canule est l'extrémité distale de l'aiguille comprise entre 0.1 et 0.5 mm, de préférence entre 0.2 et 0.4 mm,
- la canule comprend une extrémité proximale logée dans l'embase, ladite extrémité proximale étant divergente,
- le biseau de l'extrémité distale biseautée de l'aiguille présente un angle compris entre 12° et 25°, de préférence de l'ordre de 17°, et
- l'extrémité distale biseautée de l'aiguille comprend deux découpes latérales symétriques, une longueur desdites découpes latérales étant inférieure à une longueur du biseau de l'extrémité distale biseautée.

### BREVE DESCRIPTION DES DESSINS

D'autres caractéristiques, buts et avantages de la présente invention apparaîtront mieux à la lecture de la description détaillée qui va suivre, et au regard des dessins annexés donnés à titre d'exemples non limitatifs et sur lesquels :
La figure 1a est une vue en perspective d'une embase d'un exemple de réalisation d'un dispositif de perfusion ou d'injection conforme à l'invention,
La figure 1b est une vue en coupe de l'embase de la figure 1a,
La figure 2a est une vue en perspective d'une cage anti-pique d'un exemple de réalisation d'un dispositif conforme à l'invention,
La figure 2b est une vue en coupe de la cage anti-pique de la figure 2a,

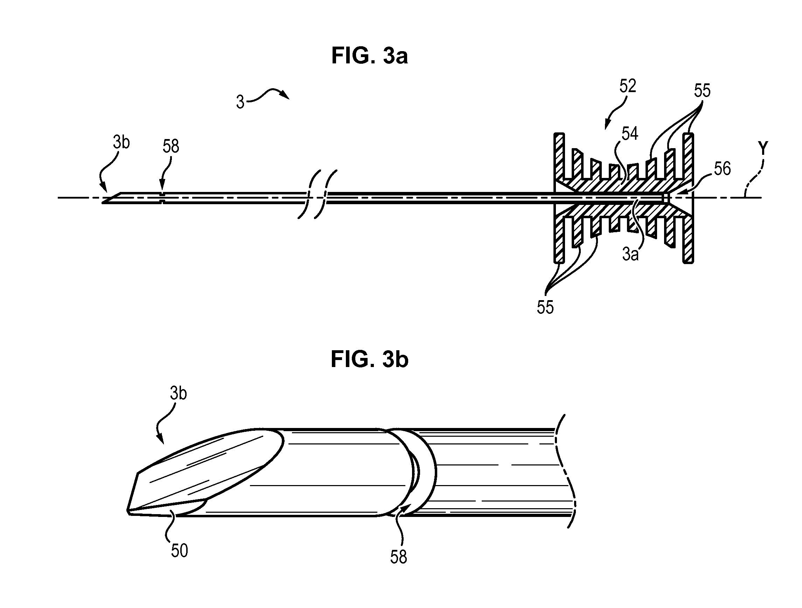

La figure 3a est une vue en coupe d'une aiguille d'un exemple de réalisation d'un dispositif conforme à l'invention,
La figure 3b est une vue en détail d'une partie de l'aiguille de la figure 3a,

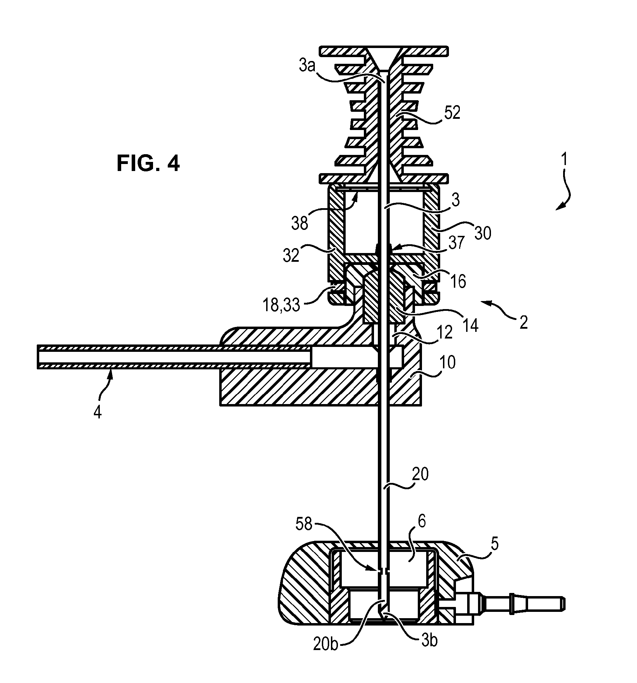

La figure 4 est une vue en coupe d'un exemple de réalisation d'un dispositif conforme à l'invention, introduit dans une chambre implantable, La figure 5 est une vue en perspective de la figure 4,
La figure 6 est une vue en perspective du dispositif de la figure 4 lors du retrait de l'aiguille,

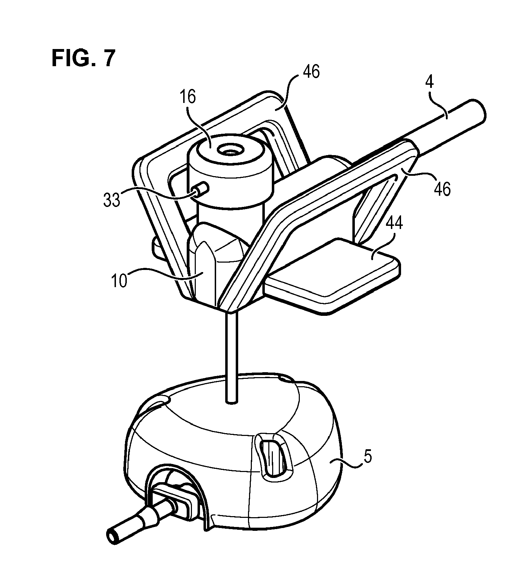

La figure 7 est une vue en perspective du dispositif de la figure 4 lors de son extraction de la chambre implantable, et

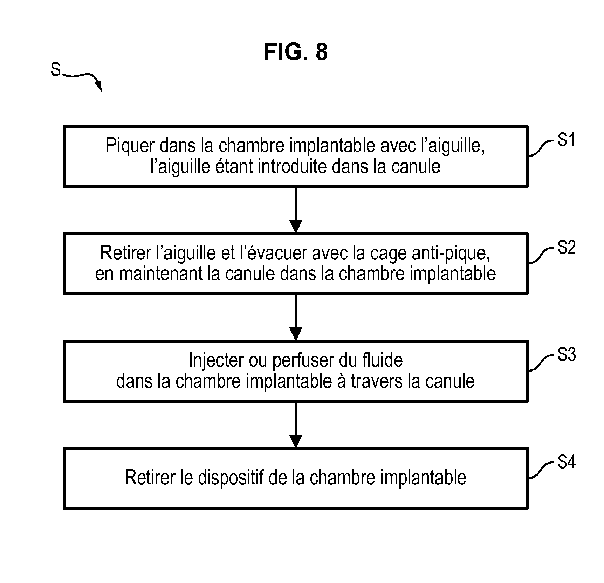

La figure 8 est un organigramme représentant différentes étapes d'un exemple de réalisation du procédé de perfusion ou d'injection sécurisé conforme à l'invention.

### DESCRIPTION DETAILLEE D'UN MODE DE REALISATION

Un dispositif 1 de perfusion ou d'injection sécurisé, notamment pour la perfusion ou l'injection d'un fluide dans une chambre implantable 5 d'un patient, va à présent être décrit en référence aux figures annexées.

Dans la suite de la description, « proximal » désignera une partie ou qui se situe à proximité de l'opérateur lorsque celui-ci utilise le dispositif 1 de l'invention et comprendra une référence indexée « a », tandis que « distal » désignera une partie qui est éloignée de l'opérateur au cours de cette utilisation et comprendra une référence indexée « b ».

Un dispositif 1 de perfusion ou d'injection sécurisé comprend une embase 2 et une aiguille 3.

L'embase 2 comprend un raccord principal 10, une canule 20 et, de manière optionnelle, une cage anti-pique 30 fixée de manière amovible sur l'embase 2 et un tube prolongateur 4. L'aiguille 3 est montée à coulissement dans l'embase 2 et dans la cage anti-pique 30 entre une première position, dans laquelle l'aiguille 3 est introduite dans la chambre implantable 5 et une deuxième position, dans laquelle l'aiguille 3 est extraite de la chambre implantable 5 et le cas échéant bloquée par la cage anti-pique 30 afin de protéger l'opérateur.

Le raccord principal 10 est configuré pour être appliqué contre la peau du patient et forme un passage principal 12 adapté pour recevoir l'aiguille 3 à coulissement suivant un axe de coulissement X.

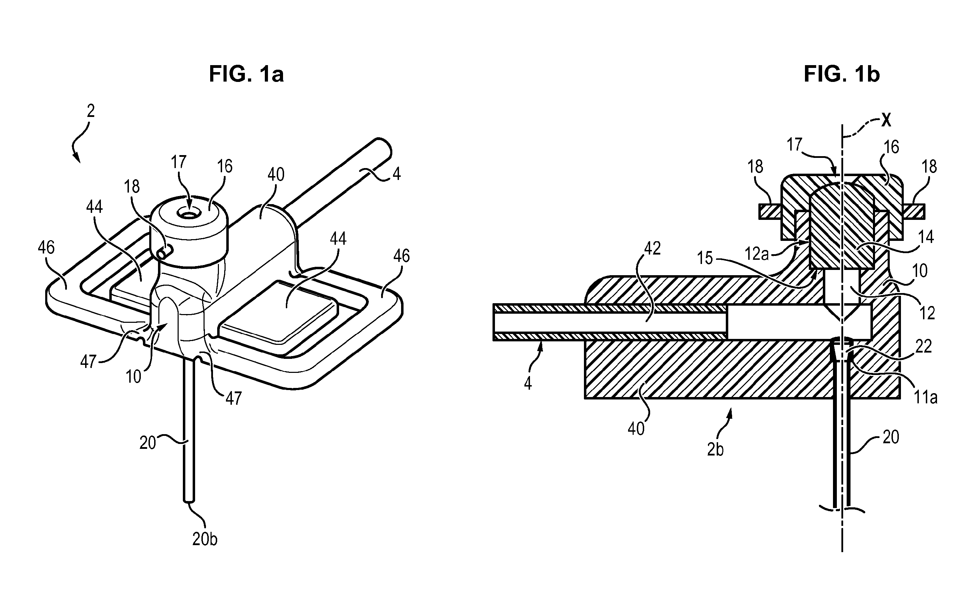

Dans l'exemple de réalisation visible sur les figures 1a et 1b, le raccord principal 10 comprend un septum 14 et un capot 16 pour étanchéifier le passage et permettre, comme nous le verrons par la suite dans la description, la fixation de la cage anti-pique 30 sur l'embase 2.

Le septum 14 est logé dans le passage principal 12, au niveau d'une partie proximale 12a de celui-ci, et est configuré pour obturer le passage principal 12. Pour cela, la section du passage principal 12 est rétrécie en partie proximale 12a de manière à former un rebord 15 contre lequel le septum 14 vient en butée.

Le capot 16 quant à lui est fixé sur le raccord principal 10 et ferme le passage principal 12. Il comprend un orifice traversant 17, coaxial avec l'axe X, à travers lequel l'aiguille 3 pénètre dans le passage principal 12. L'orifice 17 peut notamment présenter un contour chanfreiné afin de faciliter l'introduction et le guidage de l'aiguille 3 à travers le capot 16 et le raccord principal 10.

Le capot 16 est en outre configuré pour enfermer le septum 14 dans le raccord principal 10 et le comprimer dans cette position afin d'étanchéifier le passage principal 12. Pour cela, le capot 16 applique une contrainte sur la face proximale du septum 14 qui tend à plaquer le septum 14 contre le rebord 15 et à obturer ainsi le passage principal 12. Le septum 14 obture donc le passage principal 12 à tout instant, même en cas de contre-pression en provenance de l'intérieur du raccord principal 10.

Ici, le septum 14 est plein, c'est-à-dire qu'il ne présente pas de passage préformé ou pré-fendu pour l'aiguille 3. Par ailleurs, il comprend une surface proximale 14a convexe, tandis que la face en regard du capot 16 présente une cavité concave 16a complémentaire conformée pour venir en contact avec la surface convexe 14a du septum 14. De la sorte, en cas de contre-pression importante à l'intérieur du passage principal 12, le septum 14 est comprimé contre le capot 16 ce qui améliore son étanchéité.

Le septum 14 est de préférence réalisé dans un matériau résilient, par exemple en polyisoprène.

L'embase 2 et le capot 16 peuvent être obtenus par injection-moulage d'un matériau thermoplastique du type chlorure de polyvinyle (PVC). Le capot 16 est de préférence formé séparément, puis rapporté sur le raccord principal 10 et fixé sur celui-ci, par exemple par collage à l'aide d'une colle à base de Tétrahydrofurane (THF).

Le capot 16 peut en outre comprendre un système de verrouillage 18, adapté pour fixer de manière amovible la cage anti-pique 30. Ici, le système de verrouillage 18 est du type à baïonnettes, et comprend un ou plusieurs picots 18 configurés pour pénétrer dans des gorges coudées 33 complémentaires. Dans l'exemple des figures 1a et 1b, le capot 16 comprend par exemple deux picots 18 opposés s'étendant transversalement à l'axe X.

En variante, le système de verrouillage 18 pourrait également être formé directement sur le raccord principal 10.

Le capot 16 de l'embase 2 permet donc de simplifier le dispositif 1, en servant à la fois à la fixation de la cage anti-pique 30, au blocage et à la compression du septum 14 et au guidage de l'aiguille 3 dans le dispositif 1.

La canule 20 s'étend depuis l'embase 2 dans le prolongement du passage principal 12, et est configurée pour recevoir l'aiguille 3 à coulissement.

Pour cela, le passage principal 12 comprend un rétrécissement 11 débouchant en partie distale de l'embase 2. Le rétrécissement 11 est coaxial avec l'axe X, et est configuré pour recevoir la canule 20.

La canule 20 est de forme globalement cylindrique, de préférence cylindrique de révolution autour de l'axe X, et est fixée dans l'embase 2. Par exemple, la canule 20 peut comprendre une extrémité proximale divergente 22, tandis que le rétrécissement 11 peut être évasé en partie proximale 11a de manière à recevoir à et à loger l'extrémité distale divergente 22 de la canule 20. Ainsi, il suffit d'introduire la canule 20 dans le passage principal 12 (avant introduction du septum 14) par son extrémité proximale, et de la faire coulisser en force dans le passage principal 12 jusqu'à ce que son extrémité proximale divergente 22 arrive en butée contre l'évasement 11a pour fixer la canule 20 dans l'embase 2. Il n'est alors pas nécessaire de surinjecter l'embase 2 sur la canule 20, ce qui simplifie les opérations de fabrication du dispositif 1.

Afin d'assurer le blocage de la canule 20 dans l'embase 2, il est notamment possible d'accentuer la divergence de son extrémité proximale 22. On notera en outre que la partie divergente 22 de la canule 20 permet de guider l'aiguille 3 lors de son introduction dans le dispositif 1. Enfin, en cas de pression importante dans le passage principal 12, la partie divergente 22 de la canule 20 reçoit un effort de pression important qui a pour conséquence de la pousser au fond de son logement 11 a.

L'extrémité distale 20b de la canule 20 est de préférence biseautée et émoussée, afin de réduire les risques d'endommagement de la membrane 6 de la chambre implantable 5 lors de l'introduction et du retrait de la canule 20. Le biseau 20b de la canule 20 forme un angle qui peut être compris entre 5° et 20°, par exemple de l'ordre de 10°. Cet angle permet au liquide perfusé de passer entre la canule et le fond de la chambre quand la canule est en appui contre le fond, sans gêner la sortie du liquide.

Les dimensions (diamètre et longueur) de la canule 20 sont globalement similaires aux dimensions des aiguilles de Huber conventionnelles. Par exemple, la canule 20 peut présenter un calibre compris entre 14 G et 24 G (soit un diamètre compris entre 1.6 mm et 0.5 mm). La canule 20 est donc atraumatique. Par ailleurs, la canule 20 peut être réalisée en acier inoxydable, notamment un acier de nuance 316 L.

Le tube prolongateur 4 est logé dans un passage secondaire 42 de l'embase 2 et est en communication fluidique avec le passage principal 12 du raccord principal 10. Pour cela, l'embase peut par exemple comprendre un raccord secondaire 40 fixé solidairement au raccord principal 10 et définissant le passage secondaire 42, de sorte que le passage secondaire 42 débouche entre le rebord 15 et l'évasement 11a du passage principal 12.

Le raccord principal 10 et le raccord secondaire 40 peuvent être formés intégralement et en une seule pièce.

Le tube prolongateur 4 peut notamment être réalisé en matériau thermoplastique tel que du polyuréthane (PUR), et être surmoulé lors de la réalisation de l'embase 2.

Ici, le tube prolongateur 4 s'étend dans un plan sensiblement transversal à l'axe X. En variante, le tube prolongateur 4 pourrait former un angle différent de 90° avec l'axe X.

L'embase 2 peut comprendre deux plateaux de préhension 44 s'étendant depuis l'embase 2, de part et d'autre de celle-ci.

Dans la forme de réalisation illustrée sur les figures, les plateaux de préhension 44 s'étendent dans le prolongement d'une face distale 2b de l'embase 2, afin de permettre à l'opérateur de plaquer l'embase 2 contre la peau du patient lors de l'utilisation du dispositif 1, et en particulier lors de l'introduction du dispositif 1 dans la chambre implantable 5 et de l'injection ou la perfusion S d'un fluide. Les plateaux de préhension 44 améliorent ainsi la manipulation du dispositif 1, en formant des surfaces d'appui pour les doigts de l'opérateur.

L'embase 2 peut en outre comprendre des ailettes de préhension 46, qui s'étendent également de part et d'autre de l'embase 2 dans le prolongement de la face distale 2b de l'embase 2. Ici, les ailettes de préhension 46 comprennent un bord périphérique qui entoure les plateaux de préhension 44 et sont coplanaires avec ceux-ci. Les ailettes de préhension 46 présentent des affaiblissements locaux 47 au niveau de leur fixation sur l'embase 2, ce qui permet de les assouplir pour permettre à l'opérateur de les plier afin de les rapprocher l'une de l'autre pour extraire plus facilement le dispositif 1 de la chambre implantable 5.

Les plateaux de préhension 44 et les ailettes de préhension 46 peuvent par exemple être formés intégralement et en une seule pièce avec le reste de l'embase 2. Les affaiblissements locaux 47 des ailettes de préhension 46 peuvent alors comprendre un amincissement local des ailettes 46 au niveau de leur fixation.

L'aiguille 3 est pleine et présente une extrémité distale biseautée 3b, afin de faciliter son introduction dans une membrane 6 d'une chambre implantable 5. Par ailleurs, l'aiguille 3 étant pleine, elle est très résistante aux chocs et risque donc moins facilement de s'abîmer lors de son introduction dans la chambre implantable 5 qu'une aiguille 3 creuse de diamètre équivalent.

Par exemple, l'extrémité distale 3b peut comprendre un biseau présentant un angle compris entre 12° et 25°, de préférence de l'ordre de 17°. Par ailleurs, l'extrémité distale 3b peut comprendre deux découpes latérales 50 symétriques formant des back-cuts. Les découpes 50 de type back-cuts sont des affûtages généralement utilisés pour les cathéters courts afin de faciliter la pénétration des cathéters indépendamment de l'angle de ponction. Une longueur de chaque découpe 50 est de préférence inférieure à une longueur totale de la partie biseautée de l'extrémité distale 3b. La forme de l'extrémité distale 3b de l'aiguille 3 (biseau de l'ordre de 17° muni de deux découpes de type back-cut) facilite en outre l'introduction de l'aiguille 3 dans la canule 20.

L'aiguille s'étend suivant une direction principale qui définit un axe Y passant par son extrémité proximale 3a et son extrémité distale 3b. Elle peut être équipée, au niveau d'une extrémité proximale 3a opposée à l'extrémité distale 3b, d'un organe de manipulation 52. L'organe de manipulation 52 est fixé sur l'extrémité proximale 3a de l'aiguille 3 afin d'être solidaire en mouvement de celle-ci. Ici, l'organe de manipulation 52 comprend une base de forme ergonomique, symétrique en rotation autour de l'axe Y, dont une surface externe présente une concavité adaptée pour faciliter sa préhension par un opérateur. L'organe 50 peut en outre comprendre un orifice central 56, coaxial avec l'axe Y, adapté pour recevoir en force l'aiguille 3 et la bloquer en position dans l'orifice central 56 par rapport à la base 54. Par ailleurs, afin de faciliter l'insertion de l'aiguille 3 dans la base 54, l'orifice central 56 peut être évasé en partie proximale et/ou en partie distale.

La base 54 peut être obtenue par injection, par exemple en K-résine®. Par ailleurs, elle peut être formée par une série de plateaux annulaires coaxiaux 55 de diamètre externe variable afin de limiter les retassures et permettre une prise en main ergonomique. La périphérie de plateaux annulaires 55 définit alors la surface externe concave de l'organe de manipulation 52.

L'aiguille 3 étant mobile en translation à l'intérieur de la canule 20, son diamètre externe est inférieur au diamètre interne de la canule 20. Typiquement, l'ajustage en diamètre entre l'aiguille 3 et la canule 20 est de quelques centièmes de millimètres, par exemple entre 0.05 et 0.005 mm, typiquement de l'ordre de 0.02 mm.

Par ailleurs, la longueur de l'aiguille 3 et la longueur de la canule 20 sont choisies de sorte que, lorsque le dispositif 1 est assemblé et que l'aiguille 3 est amenée en butée contre l'embase 2 (qu'il s'agisse du capot 16 ou de la cage anti-pique 30, selon le mode de réalisation du dispositif 1), la distance entre la pointe de l'extrémité distale biseautée 3b de l'aiguille 3 et la pointe de l'extrémité distale biseautée 20b de la canule 20 est comprise entre 0.01 mm et 0.05 mm, de préférence de l'ordre de 0.2 mm à 0.4 mm. Ceci peut notamment être réalisé grâce à l'utilisation d'une aiguille 3 biseautée de type back-cut, dont le biseau peut être relativement court.

Dans une forme de réalisation, l'aiguille 3 comprend, à distance de son extrémité distale 3b, un organe 58 adapté pour coopérer avec un organe complémentaire de la cage anti-pique 30 afin de bloquer l'aiguille 3 dans ladite cage 30 suite à l'extraction de l'aiguille 3 de la chambre implantable 5. Comme nous le verrons dans la suite de la description, l'organe 58 peut notamment comprendre une rainure circonférentielle, coaxiale avec l'axe Y. On notera que cette forme de réalisation de l'organe 58 est tout particulièrement autorisée par le fait que l'aiguille 3 est pleine.

Optionnellement, l'embase 2 peut en outre comprendre une cage anti-pique 30.

La cage anti-pique 30 est montée de manière amovible sur l'embase 2 à l'aide du système de verrouillage 18 et comprend un corps creux 32 de forme globalement cylindrique, ici cylindrique de révolution, définissant un passage central 34 pour l'aiguille 3. Lorsque le corps creux 32 est fixé sur l'embase 2, le passage central 34 s'étend dans le prolongement du passage principal 12 afin de permettre le coulissement de l'aiguille 3. Dans la forme de réalisation illustrée sur les figures, le corps creux 32 est coaxial au raccord principal 10.

Le corps creux 32 comprend une extrémité proximale 30a et une extrémité distale 30b ouvertes, ainsi qu'un moyen de guidage 36 de l'aiguille 3. Par exemple, le moyen de guidage de l'aiguille 3 peut comprendre une paroi centrale 36 s'étendant sensiblement transversalement à l'axe X dans laquelle est formé un orifice traversant 35 coaxial avec l'axe X, configuré pour recevoir l'aiguille 3 à coulissement.

La paroi 36 peut en outre comprendre une protubérance annulaire 37 formant une douille entourant l'orifice traversant 35. La douille 37 s'étend depuis la paroi centrale 36 vers l'extrémité proximale ouverte, et permet d'améliorer le guidage de l'aiguille 3 dans l'orifice traversant 35. La douille 37 peut notamment présenter une paroi externe de forme conique afin de limiter les risques de réintroduction accidentelle de l'aiguille 3 dans l'orifice une fois ladite aiguille 3 extraite de la chambre implantable 5.

Dans la forme de réalisation illustrée sur les figures, la douille 37 est formée intégralement et en une seule pièce avec la paroi centrale 36 et le corps creux 32, par exemple par injection moulage d'un matériau plastique du type polypropylène.

Le corps creux 32 comprend des organes 33 configurés pour coopérer avec l'embase 2 afin de verrouiller la cage anti-pique 30 sur ladite embase 2. Ici, les organes comprennent par exemple deux gorges coudées 33 opposées formées dans le corps creux 32 et configurées pour recevoir et bloquer les picots 18 du système de verrouillage à baïonnettes 18, 33 du capot 16.

La cage anti-pique 30 comprend en outre un organe de retenue anti-pique 38, logé dans le passage central 34 du corps creux 32 et adapté pour venir en prise avec l'organe associé 58 de l'aiguille 3. Par exemple, l'organe de retenue peut comprendre une rondelle 38 fixée dans le corps creux 32 et configurée pour venir en prise avec la rainure circonférentielle 58 de l'aiguille 3.

La rondelle 38 est de forme globalement complémentaire des parois internes définissant le passage central 34 du corps creux 32. Ainsi, dans l'exemple de réalisation illustrée sur les figures, la rondelle 38 est de forme globalement cylindrique de révolution.

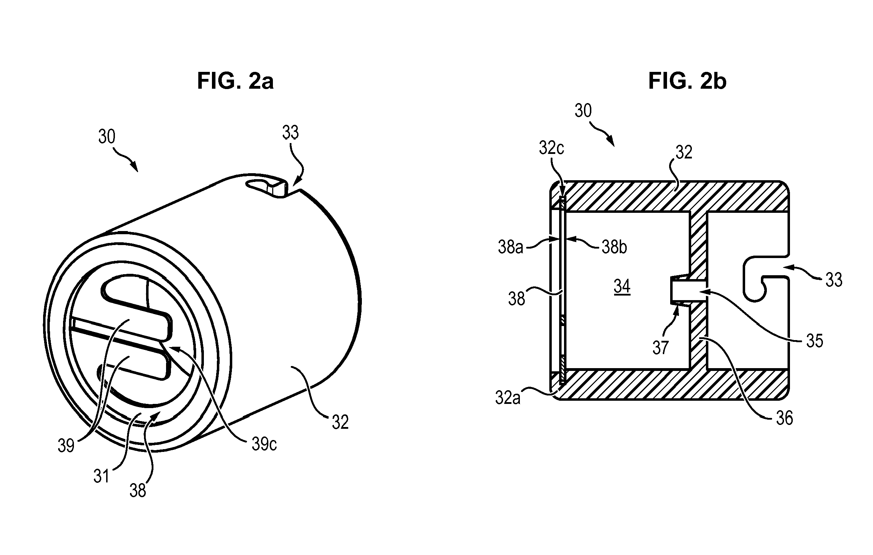

La rondelle 38 peut être plane comprendre une bordure périphérique 31, adaptée pour coopérer avec le corps creux 32 pour fixer la rondelle 38 en position, et une zone centrale 39, configurée pour coopérer avec l'aiguille 3 afin de permettre son coulissement lors de l'introduction de l'aiguille 3 dans la chambre implantable 5 et son blocage lors du retrait de l'aiguille 3 de la chambre implantable 5. Dans l'exemple des figures 2a et 2b, la zone centrale comprend deux pattes 39 s'étendant sensiblement parallèlement depuis la bordure périphérique 31 en direction du centre de la rondelle 38. De préférence, la longueur des pattes 39 entre la bordure périphérique 31 et leur extrémité libre est plus grande que le rayon de la rondelle 38, de sorte que les pattes 39 dépassent le centre de la rondelle 38. Les pattes 39 sont par ailleurs séparées par un espace 39c de largeur sensiblement inférieur au diamètre de l'aiguille 3. Ainsi, lorsque l'aiguille 3 coulisse entre les pattes 39 de la rondelle 38, lesdites pattes 39 frottent latéralement contre l'aiguille 3 avec un léger serrage.

Ainsi, lors du retrait de l'aiguille 3 de la chambre implantable 5, les pattes 39 de la rondelle 38 frottent latéralement le long de l'aiguille 3 jusqu'à venir se loger dans la rainure circonférentielle 58. L'aiguille 3 est alors bloquée en translation et ne peut plus coulisser dans le corps creux 32, ni en direction proximale ni en direction distale. Par conséquent, l'opérateur ne peut pas sortir l'aiguille de la cage anti-pique 30 ni la réintroduire dans le raccord principal 10.

De préférence, la rainure circonférentielle 58 et la rondelle 38 sont positionnées de manière à ce que, lorsqu'elles sont en prise, l'extrémité distale biseautée 3b de l'aiguille 3 se trouve dans le corps creux 32 pour limiter les risques que l'opérateur se pique. A cet effet, la rondelle 38 peut notamment être fixée au niveau de l'extrémité proximale 32a du corps creux 32, tandis que la rainure circonférentielle 58 peut être formée à une distance inférieure à la distance entre la paroi centrale 36 et la rondelle 38.

La rondelle 38 peut être réalisée dans un matériau métallique, par exemple de l'acier inoxydable de nuance 304 L, et présenter une épaisseur (suivant la direction X de coulissement de l'aiguille 3) comprise entre 0.2 mm et 0.3 mm. Dans cette forme de réalisation, les pattes 39 sont alors déformables élastiquement et autorisent ainsi un jeu qui permet à la fois le coulissement avec serrage de l'aiguille 3 lors de l'introduction de l'aiguille 3 dans la chambre implantable 5 et le blocage avec souplesse de l'aiguille 3 en venant se loger dans la rainure circonférentielle 58 de celle-ci.

La bordure périphérique 31 et les pattes 39 de la rondelle 38 peuvent être obtenues par découpage d'une plaque métallique à l'aide d'un poinçon et d'une matrice. La rondelle 38 présente alors, au niveau d'une première face 38b, des bords arrondis, et au niveau d'une deuxième face 38a, des bords plus tranchants. De préférence, la première face 38b à bords arrondis correspond à la face proximale de la rondelle 38 et est positionnée en regard de la canule 20, afin de faciliter le retrait de l'aiguille 3.

Afin de fixer la rondelle 38 dans le corps creux 32, le corps creux 32 peut notamment comprendre une gorge annulaire 32c formée dans une zone adjacente à son extrémité proximale 30a, configurée pour recevoir la zone périphérique de la rondelle 38. La hauteur de la gorge annulaire 32c est au moins égale à l'épaisseur de la rondelle 38 et de préférence légèrement plus grande, afin de ménager une liberté de mouvement de la rondelle 38 et lui permettre de se déformer élastiquement lors du coulissement de l'aiguille 3. La rondelle 38 peut notamment être alors sertie à chaud en laissant un petit jeu dans la gorge annulaire 32c du corps creux 32.

La rainure circonférentielle 58 peut notamment présenter une profondeur de l'ordre de 0.2 à 0.25 mm, et une hauteur (suivant la direction d'extension de l'aiguille 3) supérieure à l'épaisseur de la rondelle 38, typiquement de l'ordre de 0.3 à 0.4 mm pour une rondelle 38 de 0.2 mm d'épaisseur.

Les différents éléments décrits ci-dessus peuvent alors être assemblés afin de former un dispositif 1 conforme à un exemple de réalisation de l'invention.

La canule 20 peut être fixée dans l'embase 2 en l'introduisant dans le passage principal 12 à travers l'extrémité proximale, jusqu'à ce que sa partie proximale divergente 22 vienne se loger dans l'évasement 11a. Un septum 14 peut alors être introduit dans la partie proximale 34a du passage principal 12, de manière à obturer ledit passage 12, puis comprimé par le capot 16 dans cette position. Le capot 16 est alors fixé sur le raccord principal 10, par exemple à l'aide du solvant Tetrahydrofurane.

Le tube prolongateur 4 peut être soit rapporté et fixé sur l'embase 2, soit fixé par surinjection lors de la fabrication de ladite embase 2.

Optionnellement, une cage anti-pique 30 peut être verrouillée sur le raccord principal 10, par exemple à l'aide du système de verrouillage 18, 33 à baïonnettes décrit ci-avant.

Le dispositif 1 est alors assemblé et prêt à être utilisé pour perfuser ou injecter un fluide dans une chambre implantable 5.

Préalablement à l'injection ou à la perfusion du fluide, l'aiguille 3 est introduite dans l'embase 2. Pour cela, l'aiguille 3 est introduite dans le passage central 34 de la cage anti-pique 30, à travers l'orifice traversant 35 et le cas échéant la douille 37 de la paroi centrale 36, puis à travers l'orifice traversant 17 du capot 16, le septum 14 et enfin la canule 20.

On notera que, lorsque le septum 14 n'est pas pré-formé ni pré-fendu, il suffit d'appliquer un effort sur l'aiguille 3 afin de le traverser suivant l'axe X.

Dans cette configuration, l'extrémité distale biseautée de l'aiguille 3 fait saillie en dehors de la canule 20. De plus, l'axe X de l'embase 2 et l'axe Y de l'aiguille 3 sont confondus.

Le dispositif 1 peut alors être introduit dans la chambre implantable 5 suivant le procédé S de perfusion ou d'injection décrit ci-après.

Pour cela, au cours d'une première étape S1, l'opérateur saisit le dispositif 1, en maintenant l'aiguille 3 fixe par rapport à la canule 20. En particulier, l'aiguille 3 doit faire saillie de la canule 20. Pour cela, l'opérateur peut notamment amener la base de l'aiguille 3 en butée contre l'embase 2 (plus précisément contre le capot 16 ou le cas échéant la cage anti-pique 30).

L'opérateur pique alors dans la peau du patient en poussant sur l'aiguille 3 (par l'intermédiaire de son organe de manipulation 52) et l'embase 2 jusqu'à ce que l'aiguille 3 vienne en contact avec le fond de la chambre implantable 5. Le cas échéant, l'opérateur peut s'aider des plateaux de préhension 44 afin d'introduire l'aiguille 3 dans la chambre implantable 5.

Dans cette configuration, l'extrémité de l'aiguille 3 et de la canule 20 sont logées dans la chambre implantable 5. On rappelle en effet que la distance entre la pointe de l'extrémité distale biseautée 3b de l'aiguille 3 et la pointe de l'extrémité distale biseautée 20b de la canule 20 est faible, typiquement de l'ordre de 0.2 mm à 0.4 mm.

Cette étape est atraumatique pour le patient, le diamètre de la canule 20 étant sensiblement égal au diamètre habituel des aiguilles de Huber tandis que le diamètre de l'aiguille 3 est plus faible. Par ailleurs, l'aiguille 3 étant pleine, elle ne carotte pas le septum 14, ce qui réduit les risques de l'endommager. Enfin, l'ajustement (de l'ordre de quelques centièmes de millimètres) entre l'aiguille 3 et la canule 20 et l'émoussement de l'extrémité distale 20b de la canule 20 empêchent le carottage du septum 14 par la canule 20, bien que celle-ci soit creuse.

Au cours d'une deuxième étape S2, l'aiguille 3 est alors retirée du dispositif 1.

Pour cela, l'opérateur peut tirer sur l'organe de manipulation 52 de l'aiguille 3 suivant l'axe X afin d'extraire l'aiguille 3 de la chambre implantable 5 jusqu'à ce que celle-ci sorte du raccord principal 10, tout en maintenant la canule 20 en position dans la chambre implantable 5. La canule 20 peut d'ailleurs être poussée jusqu'au fond de la chambre 5 suite ou lors du retrait de l'aiguille 3.

Le cas échéant, l'opérateur peut s'aider des plateaux de préhension 44 afin de retirer l'aiguille 3 de la chambre implantable 5 sans déplacer le dispositif 1.

Lors de l'extraction de l'aiguille 3 de la chambre implantable 5, celle-ci n'abîme pas la membrane 6 puisqu'elle sort de la membrane 6 en passant au travers de la canule 20 qui reste en place dans la chambre 5. En d'autres termes, l'aiguille 3 n'entre en contact avec la membrane 6 qu'au moment de son introduction dans ladite membrane 6.

Le retrait de l'aiguille 3 de l'embase 2 obture le passage principal 12. En effet, de par sa résilience et l'effort de compression qui lui est appliqué par le capot 16, le septum 14 du dispositif 1 s'obture automatiquement lors du retrait de l'aiguille 3.

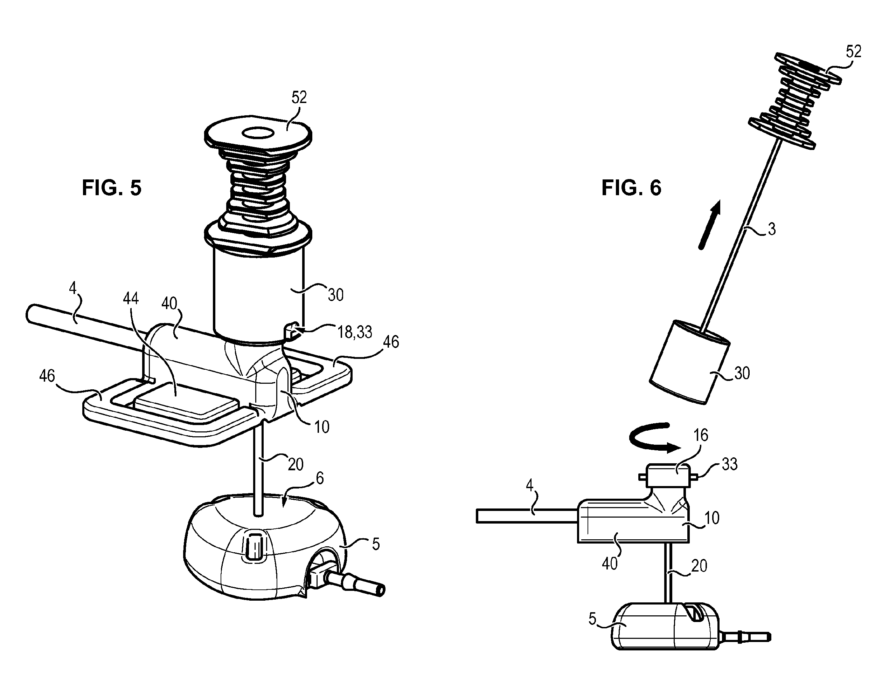

Optionnellement, lorsque le dispositif 1 comprend une cage anti-pique 30, l'opérateur peut tirer sur l'aiguille 3 sans risquer de se blesser, jusqu'à ce que l'extrémité libre biseautée 3b de l'aiguille 3 pénètre dans corps creux 32 et que l'organe de retenue anti-pique 38 bloque l'aiguille 3 en translation. Par exemple, dans le cas de la rondelle 38 et de la rainure circonférentielle 58, l'opérateur peut tirer sur l'aiguille 3 jusqu'à ce que la rainure circonférentielle 58 arrive au niveau des pattes 39 de la rondelle 38, qui viennent alors se loger par retour élastique dans la rainure 58 et bloquent le déplacement de l'aiguille 3. A ce stade, l'opérateur ne peut alors ni ré-enfoncer l'aiguille 3, ni l'extraire du corps creux 32. L'opérateur peut en revanche détacher la cage anti-pique 30, en déverrouillant le système de verrouillage 18 amovible. Ici par exemple, il suffit à l'opérateur de tourner la cage anti-pique 30 par rapport au raccord principal 10 afin de déloger les picots 18 des gorges coudées 33 correspondantes (figure 6). Ce mouvement de déverrouillage peut être effectué en toute sécurité, l'extrémité distale 3b de l'aiguille 3 étant bloquée dans le corps creux 32.

L'aiguille 3 et le cas échéant la cage anti-pique 30 peuvent alors être évacués, laissant le reste de l'embase 2 et notamment la canule 20 en position.

Au cours d'une troisième étape S3, du fluide est injecté ou perfusé dans le réseau veineux du patient, par l'intermédiaire de la chambre implantable 5. Cela est permis par la forme biseautée de l'extrémité distale 20b de la canule 20, qui permet effectivement le passage du fluide malgré le fait que la canule 20 soit en butée contre le fond de la chambre 5.

Le fluide peut être introduit dans le dispositif 1 par l'intermédiaire du tube prolongateur 4. On rappelle en effet que la partie proximale 14a du passage principal 12 est obturée et étanchéifiée par le septum 14, que sa partie distale est étanchéifiée par l'insertion en force de la partie divergente 22 de la canule 20 dans l'évasement 11a et que le tube prolongateur 4 est en communication fluidique avec le rétrécissement 11 et donc la canule 20. Le fluide introduit dans le tube prolongateur 4 passe donc successivement dans le passage secondaire 42, dans le rétrécissement 11 du passage principal 12, dans la canule 20 puis dans la chambre implantable 5.

Au cours d'une quatrième étape S4, on retire alors le dispositif 1. Pour cela, l'opérateur peut notamment plier les ailettes de préhension 46 et tirer sur l'embase 2, afin d'extraire la canule 20 de la chambre implantable 5.

Lors de son retrait, la canule 20 n'abîme pas la membrane 6 de la chambre implantable 5 puisque son extrémité distale 20b est émoussée. En conséquence, ni l'aiguille 3 ni la canule 20 ne risquent d'abîmer la membrane 6 de la chambre implantable 5, que ce soit lors de l'introduction ou du retrait du dispositif 1. A titre de comparaison, une aiguille creuse biseautée conventionnelle du type Huber, qui non seulement est plus fragile mais également épointée, a tendance à labourer la membrane 6 lors de son retrait et à la dégrader donc plus rapidement.

## Revendications

1. Dispositif de perfusion ou d'injection sécurisé (1), notamment pour la perfusion ou l'injection d'un fluide dans une chambre implantable (5), comprenant :
- une aiguille (3) pleine présentant une extrémité distale biseautée (3c), et
- une embase (2), comprenant
* un raccord principal (10), configuré pour être appliqué contre la peau du patient et formant un passage principal (12) adapté pour recevoir ladite aiguille (3) à coulissement,
* une canule (20) s'étendant depuis l'embase (2) dans le prolongement du passage principal (12), ladite canule (20) étant configurée pour recevoir l'aiguille (3) à coulissement.

2. Dispositif (1) selon la revendication 1, comprenant en outre un tube (4), fixé sur l'embase (2) et en communication fluidique avec le passage principal (12) et la canule (20).

3. Dispositif (1) selon l'une des revendications 1 ou 2, dans lequel la différence de diamètre entre la canule (20) et l'aiguille (3) est comprise entre 0.05 et 0.005 mm, par exemple de l'ordre de 0.01 mm.

4. Dispositif (1) selon l'une des revendications 1 à 3, dans lequel une extrémité distale de la canule (20) comprend un biseau émoussé formant un angle compris entre 5° et 20°, par exemple de l'ordre de 10°.

5. Dispositif (1) selon l'une des revendications 1 à 4, dans lequel le raccord principal (10) comprend en outre un septum (14) logé dans le passage principal (12) et configuré pour obturer ledit passage principal (12), et un capot (16) configuré pour comprimer le septum (14) dans le raccord principal (10).

6. Dispositif (1) selon l'une des revendications 1 à 5, dans lequel l'embase (2) comprend en outre une cage anti-pique (30), fixée de manière amovible sur une extrémité proximale (10a) du raccord principal (10), ladite cage anti-pique (30) comprenant :
- un corps creux (32) définissant un passage pour l'aiguille (3) et comprenant des organes de verrouillage amovibles (33), adaptés pour venir en prise avec des organes de verrouillage (18) associés formés sur l'embase (2), et
- un organe de retenue anti-pique (38), comprenant une rondelle (38) fixée dans le passage du corps creux (32) et adaptée pour venir en prise avec un organe (58) associé de l'aiguille (3).

7. Dispositif (1) selon la revendication 6, dans lequel l'organe associé de l'aiguille (3) comprend une rainure circonférentielle (58), et l'organe de retenue anti-pique (38) est disposé au niveau d'une extrémité proximale du corps creux (32) et comprend des pattes (69) adaptée pour venir se loger dans la rainure circonférentielle (58) et limiter le coulissement de l'aiguille (3) par rapport au corps creux (32).

8. Dispositif (1) selon l'une des revendications 6 ou 7, dans lequel l'organe de retenue (38) est en métal et est obtenu par découpage à l'aide d'un poinçon et d'une matrice de sorte que ledit organe de retenue (38) présente une première face (38b) dont des bords sont arrondis et une deuxième face (38a) dont des bords sont tranchants, la première face (38b) étant configurée pour s'étendre en regard de la canule (20).

9. Dispositif (1) selon l'une des revendications 6 à 8, dans lequel le corps creux (32) comprend en outre une paroi centrale (36), s'étendant sensiblement perpendiculairement à une direction (X) de coulissement de l'aiguille (3), ladite paroi centrale (36) présentant en son centre un orifice traversant (35) pour guider l'aiguille (3).

10. Dispositif (1) selon l'une des revendications 6 à 9, dans lequel les organes de verrouillage amovibles (18, 33) sont du type à baïonnette.

11. Dispositif (1) selon l'une des revendications 6 à 10, dans lequel l'aiguille (3) comprend, au niveau d'une extrémité proximale, un organe de manipulation (52) solidaire en mouvement de ladite aiguille (3) et adapté pour venir en butée contre l'embase (2) lorsque l'aiguille (3) est introduite dans le passage principal (12) dudit raccord principal (10).

12. Dispositif (1) selon l'une des revendications 1 à 11, dans lequel la canule (20) est configurée de sorte que, lorsque l'organe de manipulation (52) est en butée contre l'embase (2), une distance entre une extrémité distale (20b) de la canule (20) est l'extrémité distale (3b) de l'aiguille (3) comprise entre 0.1 et 0.5 mm, de préférence entre 0.2 et 0.4 mm.

13. Dispositif (1) selon l'une des revendications 1 à 12, dans lequel la canule (20) comprend une extrémité proximale (20a) logée dans l'embase (2), ladite extrémité proximale (20a) étant divergente.

14. Dispositif (1) selon l'une des revendications 1 à 13, dans lequel le biseau de l'extrémité distale biseautée (3b) de l'aiguille (3) présente un angle compris entre 12° et 25°, de préférence de l'ordre de 17°.

15. Dispositif (1) selon l'une des revendications 1 à 14 dans lequel l'extrémité distale biseautée (3b) de l'aiguille (3) comprend deux découpes latérales (50) symétriques, une longueur desdites découpes latérales (50) étant inférieure à une longueur du biseau de l'extrémité distale biseautée (3b).
